# EUROPEAN PATENT APPLICATION

(11) **EP 4 335 429 A1**
(43) Date of publication of application: **13.03.2024**
(21) Application number: 23192911.8
(22) Date of filing: 23.08.2023
(51) Int. Cl.: A61K 6/17, C03C 12/00, A61K 6/889, A61K 6/836

(54) **METHOD FOR PRODUCING ACID-REACTIVE GLASS POWDER, ACID-REACTIVE GLASS POWDER OBTAINED BY THE PRODUCTION METHOD, AND DENTAL GLASS IONOMER CEMENT COMPOSITION CONTAINING THE SAME**

(30) Priority: 05.09.2022 JP 2022140844
(71) Applicant: Kabushiki Kaisha Shofu, Kyoto-shi Kyoto 605-0983 (JP)
(72) Inventor: SAKAMOTO, Shuji, Kyoto, 605-0983 (JP); ITAGAKI, Takuma, Kyoto, 605-0983 (JP); NISHIMURA, Manami, Kyoto, 605-0983 (JP); KIMOTO, Katsuya, Kyoto, 605-0983 (JP)
(74) Representative: Eisenführ Speiser

(57) **Abstract**

Provided are a method for producing an acid-reactive glass powder that, when used for a dental glass ionomer cement composition, has good mixing property and a sufficient working time and simultaneously can make the dental glass ionomer cement composition exhibit superior mechanical properties, an acid-reactive glass powder obtainable by the production method, and a dental glass ionomer cement composition comprising the acid-reactive glass powder. More specifically, provided are a method for producing an acid-reactive glass powder characterized in that an acid-reactive glass powder having a range of 2 to 10 µm of a 50% particle size is obtainable by pulverizing a large-particle-size acid-reactive glass powder having a range of 250 to 3000 µm of a 50% particle size in a state where a small-particle-size acid-reactive glass powder having a range of 1 to 5 µm of a 50% particle size coexists; an acid-reactive glass powder obtained by the production method; and a dental glass ionomer cement composition comprising the acid-reactive glass powder.

## Description

### TECHNICAL FIELD

The present invention relates to a method for producing an acid-reactive glass powder suitable for a glass ionomer cement for dental filling and/or a glass ionomer cement for dental luting and the like, an acid-reactive glass powder obtained by the production method, and a dental glass ionomer cement composition comprising the acid-reactive glass powder.

### BACKGROUND ART

In dental clinical practice, in order to aesthetically and functionally recover a tooth partially damaged in its form by dental caries, fracture, or the like, direct restoration in which a filling material is filled into the tooth, or indirect restoration in which a dental prosthesis equipment is bonded and/or adhered to the tooth using a luting material is performed. One of typical filling materials and luting materials may be a dental glass ionomer cement that is set only through an acid-base reaction, or a resin modified dental glass ionomer cement that is set/cured through an acid-base reaction and an accompanying polymerization reaction.

The dental glass ionomer cement is generally provided in a form in which the dental glass ionomer cement is separated into a powder comprising an acid-reactive glass powder as a main component and a liquid comprising a polyalkenoic acid and water as main components, and is used by mixing the powder and the liquid immediately before use. Such a dental glass ionomer cement composition is disclosed, for example, in Patent Documents 1 to 3.

### Related Art Documents

Patent Documents
Patent Document 1: JP H1-308853 A
Patent Document 2: JP 2008-519749 A
Patent Document 3: WO 2019/230309 A

### SUMMARY

### Problems to be Solved by the Invention

A conventional dental glass ionomer cement composition is superior in mechanical properties, but the conventional dental glass ionomer cement composition tends to have deteriorated mixing property with a liquid or have a shortened working time when the particle size of the acid-reactive glass powder as a main component is small. On the other hand, when the particle size of the acid-reactive glass powder is large, the dental glass ionomer cement composition has good mixing property and a sufficient working time, but tends to have deteriorated mechanical properties. In a case in which an acid-reactive glass powder having a small particle size and an acid-reactive glass powder having a large particle size are separately produced and then simultaneously incorporated in a dental glass ionomer cement composition, it is difficult to make the dental glass ionomer cement composition have all of desired mechanical properties, mixing property, and working time in a well-balanced manner.

In view of such circumstances, an object of the present invention is to provide a method for producing an acid-reactive glass powder that has desired mixing property and working time and exhibits desired mechanical properties when used in a dental glass ionomer cement composition, an acid-reactive glass powder obtained by the production method, and a dental glass ionomer cement composition containing the acid-reactive glass powder.

### Solutions to the Problems

As a result of intensive studies to solve the above problems, the present inventors have surprisingly and unexpectedly found that the above problems can be overcome by pulverizing a large-particle-size acid-reactive glass powder in a state where a small-particle-size acid-reactive glass powder coexists, and have accomplished the present invention.

More specifically, the above object is achieved by the following embodiments. That is, the present invention encompasses a method for producing an acid-reactive glass powder characterized in that an acid-reactive glass powder having a range of 2 to 10 µm of a 50% particle size is obtained by pulverizing a large-particle-size acid-reactive glass powder having a range of 250 to 3000 µm of a 50% particle size (i.e., having a 50% particle size in a range of 250 to 3000 µm) in a state where a small-particle-size acid-reactive glass powder having a range of 1 to 5 µm of a 50% particle size (i.e., having a 50% particle size in a range of 1 to 5 µm) coexists with the large-particle-size acid-reactive glass powder; an acid-reactive glass powder obtained by the production method; and a dental glass ionomer cement composition comprising the acid-reactive glass powder.

In one possible embodiment, the particle size distribution of the acid-reactive glass powder at a point in time after the pulverization has a first peak or shoulder (P1) in the range of 1 to 5 µm and a second peak or shoulder (P2) in the range of 3 to 20 µm, and
wherein particle sizes of the first peak or shoulder (P1) and the second peak or shoulder (P2) have a relationship of P1 < P2, and a difference between the particle sizes of the first peak or shoulder (P1) and the second peak or shoulder (P2) is 2 µm or more.

In another possible embodiment, the particle size distribution of the acid-reactive glass powder at a point in time after the pulverization has a first peak (P1) in the range of 1 to 5 µm and a second peak or shoulder (P2) in the range of 3 to 20 µm, and wherein particle sizes of the first peak (P1) and the second peak or shoulder (P2) have a relationship of P1 < P2, a difference between the particle sizes of the first peak or shoulder (P1) and the second peak or shoulder (P2) is 2 µm or more, and wherein a ratio (P1/P2) of a frequency of the first peak (P1) to a frequency of the second peak or shoulder (P2) is in the range of 1.0 to 5.0, preferably in the range of 1.1 to 4.0, and more preferably in the range of 1.2 to 3.0.

In the particle size distribution of the acid-reactive glass powder at a point in time after the pulverization, the difference between the 10% particle size and the 90% particle size may be in the range of 9 to 25 µm.

The dental glass ionomer cement composition comprising the acid-reactive glass powder after the pulverization may comprise:
60.0 to 80.0% by mass of an acid-reactive glass powder;
5.0 to 25.0% by mass of a polyalkenoic acid; and
10.0 to 25.0% by mass of water.

### Effects of the Invention

According to the method for producing an acid-reactive glass powder of the present invention, it is possible to produce an acid-reactive glass powder that, when used for a dental glass ionomer cement composition, has good mixing property and a sufficient working time and can make the dental glass ionomer cement composition exhibit superior mechanical properties.

### BRIEF DESCRIPTION OF DRAWINGS

Fig. 1 illustrates a schematic diagram for explaining a "peak" in a particle size distribution;
Fig. 2 illustrates a schematic diagram for explaining a "first shoulder" in a particle size distribution;
Fig. 3 illustrates a schematic diagram for explaining a "second shoulder" in a particle size distribution; and
Fig. 4 illustrates a schematic diagram for explaining a typical example in a particle size distribution.

### DETAILED DESCRIPTION

The present invention is described in detail below. In the present specification, a numerical range "X to Y" means a numerical range including X as a lower limit value and Y as an upper limit value unless otherwise specified with terms such as "less than" or "more than/larger than" attached.

In the present specification, the term "dental glass ionomer cement" means a dental glass ionomer cement in which no compound having a polymerizable group, such as polymerizable monomer, oligomer having a polymerizable group and/or polymer having a polymerizable group, is blended with the intention of causing curing through a polymerization reaction and which is set mainly through an acid-base reaction that occurs between an acid-reactive glass powder and a polyalkenoic acid in the presence of water.

The terms "10% particle size", "50% particle size", and "90% particle size" mean particle sizes at which, in a particle size distribution on volume basis measured using a laser diffraction-scattering particle size analyzer or the like, the values integrated from the smaller particle size side are 10%, 50%, and 90%, respectively. In the particle size distribution dealt in the present invention, the vertical axis represents the appearance frequency, the horizontal axis represents the particle size, and the horizontal axis is in the common logarithm scale.

The term "peak" means a point at which the appearance frequency is the local maximum value in the particle size distribution on volume basis, and will be described in detail later.

The term "shoulder" means a point at which the rate of change of the differentiated value is minimum in a particle size distribution on volume basis, the point being present on the right side of an arbitrary peak, and will be described in detail later.

In the method for producing an acid-reactive glass powder of the present invention, it is an essential condition to pulverize a large-particle-size acid-reactive glass powder having a range of 250 to 3000 µm of a 50% particle size in a state where a small-particle-size acid-reactive glass powder having a range of 1 to 5 µm of a 50% particle size coexists (i.e., is made to coexist), and thereby obtain an acid-reactive glass powder having a range of 2 to 10 µm of a 50% particle size.

The term "state where a small-particle-size acid-reactive glass powder coexists" includes a state where a small-particle-size acid-reactive glass powder has been added to a large-particle-size acid-reactive glass powder and the small-particle-size acid-reactive glass powder coexists therewith, a state where a large-particle-size acid-reactive glass powder has been added to a small-particle-size acid-reactive glass powder and the small-particle-size acid-reactive glass powder coexists therewith, and a state where a "small-particle-size acid-reactive glass powder" and a "large-particle-size acid-reactive glass powder" have been separately prepared or purchased and introduced into a separately prepared by pulverizing machine to coexist with each other.

When the acid-reactive glass powder produced under these conditions is used in a dental glass ionomer cement, the dental glass ionomer cement can exhibit superior mechanical properties while having good mixing property and a sufficient working time.

If a small-particle-size acid-reactive glass powder having a 50% particle size of less than 1 µm is used in the aforementioned method for producing an acid-reactive glass powder, the acid-reactive glass powder after the pulverization has remarkably high reactivity with a polyalkenoic acid. Owing to this, when the acid-reactive glass powder is used in a dental glass ionomer cement composition, the dental glass ionomer cement composition may have deteriorated mixing property or may have a shortened working time.

If a small-particle-size acid-reactive glass powder having a small particle size having a 50% particle size of more than 5 µm is used in the aforementioned method for producing an acid-reactive glass powder, a dental glass ionomer cement comprising the acid-reactive glass powder after the pulverization may have deteriorated mechanical properties.

If a large-particle-size acid-reactive glass powder having a 50% particle size of less than 250 µm is used in the aforementioned method for producing an acid-reactive glass powder, a dental glass ionomer cement comprising the acid-reactive glass powder after the pulverization may have deteriorated mixing property or a shortened working time.

If a large-particle-size acid-reactive glass powder having a 50% particle size of more than 3000 µm is used in the aforementioned method for producing an acid-reactive glass powder, a dental glass ionomer cement comprising the acid-reactive glass powder after the pulverization may have deteriorated mechanical properties or may have deteriorated mixing property.

When the acid-reactive glass powder at a point in time after the pulverization has a 50% particle size of less than 2 µm in the aforementioned method for producing an acid-reactive glass powder, a dental glass ionomer cement comprising the acid-reactive glass powder may have deteriorated mixing property or may have a shortened working time.

When the acid-reactive glass powder at a point in time after the pulverization has a 50% particle size of more than 10 µm is used in the aforementioned method for producing an acid-reactive glass powder, a dental glass ionomer cement comprising the acid-reactive glass powder may have deteriorated mechanical properties.

In the aforementioned method for producing an acid-reactive glass powder, the pulverization may be carried out such that the particle size distribution of the acid-reactive glass powder at a point in time after the pulverization has a first peak or shoulder (P1) in the range of 1 to 5 µm and a second peak or shoulder (P2) in the range of 3 to 20 µm, the particle sizes of the first peak or shoulder (P1) and the second peak or shoulder (P2) are in the relationship of P1 < P2, and the difference between the particle sizes of the first peak or shoulder (P1) and the second peak or shoulder (P2) is 2 µm or more; and the pulverization also may be carried out such that the difference between those particle sizes is 2 µm or more and 20 µm or less, 2 µm or more and 15 µm or less, or 2 µm or more and 10 µm or less. When the particle size distribution of the acid-reactive glass powder at a point in time after the pulverization fulfills the above conditions, a dental glass ionomer cement comprising the acid-reactive glass powder easily exhibits good mixing property, a sufficient working time, and superior mechanical properties in a more balanced manner.

In the aforementioned method for producing an acid-reactive glass powder, it is preferable to perform the pulverization such that the particle size distribution of the acid-reactive glass powder at a point in time after the pulverization has a first peak (P1) in the range of 1 to 5 µm and a second peak or shoulder (P2) in the range of 3 to 20 µm, the particle sizes of the first peak (P1) and the second peak or shoulder (P2) are in the relationship of P1 < P2, the difference between the particle sizes of the first peak or shoulder (P1) and the second peak or shoulder (P2) is 2 µm or more, and the ratio (P1/P2) of the frequency of the first peak (P1) to the frequency of the second peak or shoulder (P2) is in the range of 1.0 to 5.0. When the ratio (P1/P2) of the frequencies is 1.0 to 5.0, a dental glass ionomer cement comprising the acid-reactive glass powder easily exhibits better mechanical properties, or easily exhibits better mixing property and a longer working time. In the particle size distribution, the frequency of P1 is typically higher than the frequency of P2.

In the aforementioned method for producing an acid-reactive glass powder, it is preferable to perform the pulverization such that the difference between the 10% particle size and the 90% particle size in the particle size distribution of the acid-reactive glass powder after the pulverization is in the range of 9 to 25 µm. When the difference in particle size is 9 to 25 µm, a dental glass ionomer cement comprising the acid-reactive glass powder more easily exhibits good mixing property, a sufficient working time, and superior mechanical properties in a more balanced manner. Herein, a longer working time is preferred because it can afford a margin in the treatment work in the oral cavity of a patient. The working time is preferably 1.5 minutes or more, more preferably 2 minutes or more, and still more preferably 2.5 minutes or more. In addition, a dental glass ionomer cement preferably has better mechanical properties because such a dental glass ionomer cement can be used for a long period of time without causing problems such as falling off and fracture in the oral cavity of a patient. The mechanical properties, expressed by compressive strength, are preferably 180 MPa or more, more preferably 200 MPa or more, and still more preferably 230 MPa or more.

The "particle size distribution" can be measured using, for example, a laser diffraction-scattering particle size analyzer, and specifically, it is measured by a laser diffraction particle size analyzer (Microtrack MT3300EXII: manufactured by Microtrack Bell Corp.) equipped with a wet disperser.

As to the measurement conditions, typically, a dispersion of a test substance in ion-exchanged water is prepared at room temperature (23°C) and atmospheric pressure, and this dispersion is measured by setting the number of measurements: 2 times/average, the measurement time: 30 seconds, the particle refractive index: 1.55, the particle shape: non-spherical, the sensitivity setting: standard, the distribution expression: volume, the upper limit of measurement: 2000 µm, the lower limit of measurement: 0.021 µm, the particle transparency: transparent, and the solvent refractive index: 1.33.

Accordingly, the "10% particle size" typically means a particle size at which the value integrated from a smaller particle size side is 10% in a particle size distribution on volume basis measured using a laser diffraction-scattering particle size analyzer or the like, specifically a laser diffraction particle size analyzer (Microtrack MT3300EXII: manufactured by Microtrack Bell Corp.) equipped with a wet disperser, where the vertical axis represents the appearance frequency, the horizontal axis represents the particle size, and the horizontal axis is in the common logarithm scale.

In addition, the "50% particle size" typically means a particle size at which the value integrated from a smaller particle size side is 50% in a particle size distribution on volume basis measured using a laser diffraction-scattering particle size analyzer or the like, specifically a laser diffraction particle size analyzer (Microtrack MT3300EXII: manufactured by Microtrack Bell Corp.) equipped with a wet disperser, where the vertical axis represents the appearance frequency, the horizontal axis represents the particle size, and the horizontal axis is in the common logarithm scale.

The "90% particle size" typically means a particle size at which the value integrated from a smaller particle size side is 90% in a particle size distribution on volume basis measured likewise using a laser diffraction-scattering particle size analyzer where the vertical axis represents the appearance frequency, the horizontal axis represents the particle size, and the horizontal axis is in the common logarithm scale.

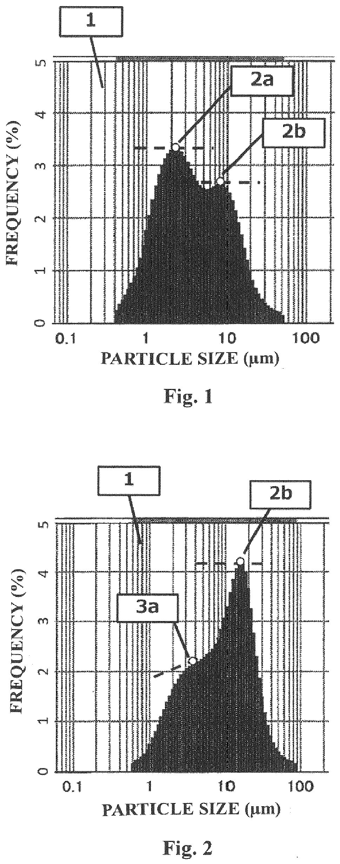

In addition, the "first peak" means a point at which the appearance frequency located on a smaller and smaller particle size side is a local maximum value in the particle size distribution on volume basis measured using the laser diffraction-scattering particle size analyzer, and for example, the "first peak" is shown as the left side peak 2a in Fig. 1.

Similarly, the "second peak" means a point at which the appearance frequency located on a larger and larger particle size side is a local maximum value in the particle size distribution, and for example, the "second peak" is shown as the right side peak 2b in Fig. 1.

The "first shoulder" means a point at which the rate of change of the differentiated value located on the left side of an arbitrary peak is minimum in the particle size distribution on volume basis measured by the laser diffraction-scattering particle size analyzer, and for example, the "first shoulder" is shown as a shoulder 3a located on the left side of the second peak 2b in Fig. 2.

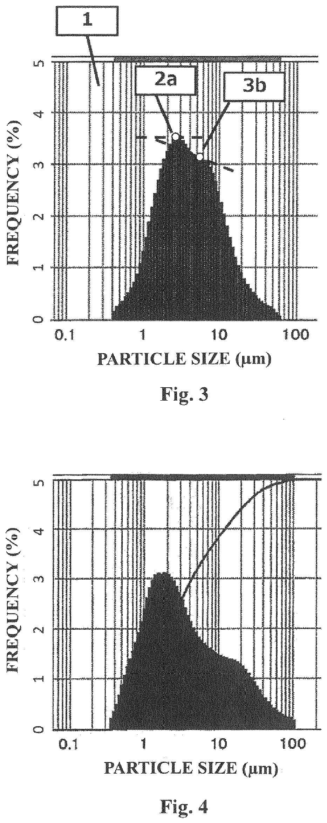

The "second shoulder" means a point at which the rate of change of the differentiated value located on the right side of an arbitrary peak is minimum in the particle size distribution on volume basis measured by the laser diffraction-scattering particle size analyzer, and for example, the "second shoulder" is shown as a shoulder 3b located on the right side of the first peak 2a in Fig. 3.

Next, the glass constituents of the acid-reactive glass powders to be used for the method for producing an acid-reactive glass powder of the present invention, and the methods for obtaining the small-particle-size acid-reactive glass powder and the large-particle-size acid-reactive glass powder are described.

The glass constituents of the acid-reactive glass powders to be used in the method for producing an acid-reactive glass powder of the present invention need to contain an acid-reactive element such as a metal element, and fluorine. When an acid-reactive element is contained as a glass constituent, an acid-base reaction with an acidic group of a polyalkenoic acid proceeds in the presence of water. Examples of the acid-reactive element specifically include, but are not limited to, sodium, potassium, calcium, strontium, barium, lanthanum, aluminum, and/or zinc. One or two or more of these acid-reactive elements may be contained, and the content thereof is not particularly limited.

Further, in order to impart an X-ray imaging property to the dental glass ionomer cement composition of the present invention, it is preferable that the glass constituent contains an X-ray radiopaque element. Examples of the X-ray radiopaque element specifically include, but are not limited to, strontium, lanthanum, zirconium, titanium, yttrium, ytterbium, tantalum, tin, tellurium, tungsten and/or bismuth. In addition, other elements contained in the glass constituent are not particularly limited, and various elements may be contained.

Examples of the acid-reactive glass as a raw material of the acid-reactive glass powder include, but are not limited to, aluminosilicate glass, borosilicate glass, aluminoborate glass, boroaluminosilicate glass, phosphate glass, borate glass, and/or silica glass containing the above-described acid-reactive element, fluorine, and X-ray radiopaque element.

In addition, the method for producing the acid-reactive glass as a raw material of the acid-reactive glass powder is not particularly limited, and an acid-reactive glass produced using any production method such as a melting method, a gas phase method, and a sol-gel method can be used without any problems. Among them, it is preferable to use an acid-reactive glass produced by a melting method or a sol-gel method, in which the type and/or content of an element can be easily controlled.

As the acid-reactive glass powder to be used in the method for producing an acid-reactive glass powder of the present invention, a plurality of types having different glass constituents may be used in combination. For example, the small-particle-size acid-reactive glass powder having a range of 1 to 5 µm of a 50% particle size and the large-particle-size acid-reactive glass powder having a range of 250 to 3000 µm of a 50% particle size may have different glass constituents, and a plurality of types of large-particle-size acid-reactive glass powders having different glass constituents may be pulverized in a state where a plurality of types of small-particle-size acid-reactive glass powders having different glass constituents coexist with the plurality of types of large-particle-size acid-reactive glass powders. In other words, the large-particle-size acid-reactive glass powder and the small-particle-size acid-reactive glass powder may coexist with each other or combined with each other, and then pulverized.

In addition, the "small particle" of the small-particle-size acid-reactive glass powder and the "large particle" of the large-particle-size acid-reactive glass powder merely indicate comparison of the relative particle sizes of these acid-reactive glass powders, and these terms themselves are not necessarily intended to mean particle sizes in specific ranges.

The small-particle-size acid-reactive glass powder having a range of 1 to 5 µm of a 50% particle size and the large-particle-size acid-reactive glass powder having a range of 250 to 3000 µm of a 50% particle size, which are used in the method for producing an acid-reactive glass powder of the present invention, can be obtained by appropriately selecting a pulverizing method, a secondary crusher, and a pulverizing machine suitable for the respective desired particle sizes and pulverizing arbitrary raw feeds for pulverizing composed of the acid-reactive glass. For example, it is recommended that an acid-reactive glass powder having a range of 250 to 3000 µm of a 50% particle size is used for the preparation of the small-particle-size acid-reactive glass powder having a range of 1 to 5 µm of a 50% particle size and an acid-reactive glass powder having a range of 0.5 mm to 30 mm of a 50% particle size is used for the preparation of the large-particle-size acid-reactive glass powder having a range of 250 to 3000 µm of a 50% particle size. The "small-particle-size acid-reactive glass powder" may be prepared by any method. Typically, the small-particle-size acid-reactive glass powder having a range of 1 to 5 µm of a 50% particle size can be obtained by pulverizing an acid-reactive glass powder having an initial 50% particle size in the range of 250 to 3000 µm under atmospheric pressure at room temperature (23°C) for 5 to 72 hours using a ball mill (BM-50: manufactured by Chukoh Seiki Co., Ltd.) or a vibration mill (MB-100: manufactured by Chuo Kakohki Co., Ltd.). The initial "acid-reactive glass powder having an initial 50% particle size in the range of 250 to 3000 µm" can be obtained by pulverizing the initial acid-reactive glass powder having a range of 0.5 mm to 30 mm of a 50% particle size. Similarly, the "large-particle-size acid-reactive glass powder" may also be prepared by any method. Typically, the large-particle-size acid-reactive glass powder having a range of 250 to 3000 µm of a 50% particle size can be obtained by pulverizing an acid-reactive glass powder having an initial 50% particle size in the range of 0.5 mm to 30 mm µm under atmospheric pressure at room temperature (23°C) using a roll crusher (Double Roll Crusher 1022-AFS: manufactured by Yoshida Seisakusho Co., Ltd.) or a cage mill (IB4-18H: manufactured by Kansai Matec Co., Ltd.).

As the small-particle-size acid-reactive glass powder and the large-particle-size acid-reactive glass powder, a commercially available acid-reactive glass powder pulverized to a desired particle size in advance may be used without any problems.

Although not particularly limited, the facility for producing the large-particle-size acid-reactive glass may be a "continuous type", and the facility for producing the small-particle-size acid-reactive glass may be a "batch type".

As the pulverizing method, either a wet method or a dry method may be used. The secondary crusher that grinds or pulverizes a pulverizing raw feed having a particle size in the order of several tens mm to one having a particle size in the order ranging from several mm to several hundreds µm is suitable for obtaining a large-particle-size acid-reactive glass powder, and specific examples thereof include, but are not limited to, an automated mortar and a roll crusher. In addition, the pulverizing machine that grinds or pulverizes a pulverizing raw feed having a particle size of several tens mm or less to one having a particle size of several hundred µm or less is suitable for obtaining a small-particle-size acid-reactive glass powder, and specific examples thereof include, but are not limited to, a high-speed revolution mill such as a hammer mill or a turbo mill, a container driven-type mill such as a ball mill, a planetary mill or a vibration mill, a medium stirrer mill such as an attritor or a bead mill, and a jet mill. These may be used not only alone but also in combination of two or more types.

Examples of such a combination include, but are not limited to, a combination of pulverizing an arbitrary raw feed for pulverizing composed of an acid-reactive glass is with any one of the aforementioned pulverizing machines to obtain a small-particle-size acid-reactive glass powder; and then, without taking the resulting small-particle-size acid-reactive glass powder out of the pulverizing machine, further feeding thereto a large-particle-size acid-reactive glass powder obtained with a secondary crusher in advance, followed by pulverizing, and a combination of obtaining a small-particle-size acid-reactive glass powder in the same manner as described above; then transferring the resulting small-particle-size acid-reactive glass powder to another pulverizing machine; and further feeding, to the pulverizing machine, a large-particle-size acid-reactive glass powder obtained with a secondary crusher in advance, followed by pulverizing. Of them, in consideration of workability and manufacturing cost, it is more preferable to use the former combination.

Next, other conditions in the method for producing an acid-reactive glass powder of the present invention will be described.

In the method for producing an acid-reactive glass powder of the present invention, the mixing ratio of the small-particle-size acid-reactive glass powder having a range of 1 to 5 µm of a 50% particle size to the large-particle-size acid-reactive glass powder having a range of 250 to 3000 µm of a 50% particle size is not particularly limited, but in order to easily exhibit the advantages of the present invention, the mixing ratio of the small-particle-size acid-reactive glass powder : the large-particle-size acid-reactive glass powder is preferably 90 : 10 to 10 : 90, and more preferably 80 : 20 to 20 : 80 by mass ratio. When compressive strength is regarded as important, the mixing proportion of the small-particle-size acid-reactive glass powder may be increased, and where the total mass of the small-particle-size acid-reactive glass powder and the large-particle-size acid-reactive glass powder is 100%, the small-particle-size acid-reactive glass powder may be contained in an amount of more than 50% by mass. Specifically, the mixing ratio may be in the range of 90 : 10 to more than 50 : less than 50 by mass ratio, and for example, may be in the range of 80 : 20 to more than 50 : less than 50 or in the range of 70 : 30 to more than 50 : less than 50 by mass ratio. When mixing property is regarded as important, the mixing proportion of the large-particle-size acid-reactive glass powder may be increased, and where the total mass of the small-particle-size and large-particle-size acid-reactive glass powders is 100%, the large-particle-size acid-reactive glass powder may be contained in an amount of more than 50% by mass. Specifically, the mixing ratio may be in the range of 10 : 90 to less than 50 : more than 50 by mass ratio, for example, in the range of 20 : 80 to less than 50 : more than 50 or in the range of 30 : 70 to less than 50 : more than 50 by mass ratio. From the viewpoint of the balance between the compressive strength and the mixing property, the mixing ratio may be 50 : 50 by mass ratio.

In the method for producing an acid-reactive glass powder of the present invention, the pulverizing time is not particularly limited. That is, the "time for subjecting a large-particle-size acid-reactive glass powder having a range of 250 to 3000 µm of a 50% particle size to pulverizing in a state where a small-particle-size acid-reactive glass powder having a range of 1 to 5 µm of a 50% particle size coexists" is not particularly limited. For example, depending on the type of the acid-reactive glass powder to be pulverized, the pulverizing method, and the capacity of the secondary crusher or the pulverizing machine, and depending on the type and the size of the pulverizing medium to be used, the pulverizing time can be appropriately adjusted through sampling the glass powder at arbitrary times and measuring the particle size. Although it is not intended to limit the present invention, the pulverizing time may be 1 to 50 hours, for example, 1 to 10 hours, 2 to 6 hours, 1 to 24 hours, or the like.

In the method for producing an acid-reactive glass powder of the present invention, the temperature, pressure and the like for the pulverization are also not particularly limited. Typically, the pulverization may be performed at room temperature and under atmospheric pressure. That is, the "temperature and pressure at which a large-particle-size acid-reactive glass powder having a range of 250 to 3000 µm of a 50% particle size is subjected to pulverizing in a state where a small-particle-size acid-reactive glass powder having a range of 1 to 5 µm of a 50% particle size coexists" may be room temperature and atmospheric pressure (for example, 1 atm). The term "room temperature" as used herein refers to a temperature of an environment the temperature of which is not artificially changed through heating, cooling, or the like by a person skilled in the art (for example, ambient temperature), and typically means a temperature of 5 to 40°C, for example, 10 to 30°C or 15 to 25°C.

Next, the surface treatment and the like of the acid-reactive glass powder obtained by the method for producing an acid-reactive glass powder of the present invention will be described.

In order to adjust the handling, setting property, mechanical properties, and the like of the dental glass ionomer cement composition containing an acid-reactive glass powder obtained by the production method of the present invention, the acid-reactive glass powder may be, without being accompanied by any problems, subjected to an optional treatment, such as surface treatment using a surface treatment agent, heat treatment, or aggregation treatment in a liquid phase, a gas phase or the like, as long as an acid-base reaction with a polyalkenoic acid is not adversely affected. These optional treatments may be performed alone or in combination of several types, and the order of performing the respective treatments is not particularly limited. Among them, surface treatment using a surface treatment agent or heat treatment is suitable because various properties are easily controlled and it is superior in productivity.

Examples of the surface treatment of the acid-reactive glass powder using a surface treatment agent specifically include washing with an acid such as phosphoric acid or acetic acid, surface treatment with an acidic compound such as tartaric acid or polycarboxylic acid, surface treatment with a fluoride such as aluminum fluoride, and surface treatment with a silane compound such as γ-mercaptopropyltrimethoxysilane, tetramethoxysilane, tetraethoxysilane, a partially hydrolyzed oligomer of tetramethoxysilane and/or a partially hydrolyzed oligomer of tetraethoxysilane. The surface treatment method is not limited to those described above, and these surface treatment methods can be used alone or in combination.

Examples of the heat treatment method for the acid-reactive glass powder specifically include a treatment method of heating the acid-reactive glass powder in the range of 200°C to 800°C for 1 hour to 72 hours using an electric furnace or the like. The heat treatment method is not limited to those described above, and single treatment, multistage treatment, or the like can also be employed as the treatment process.

Next, a dental glass ionomer cement composition comprising the acid-reactive glass powder obtained by the production method of the present invention is described.

When the acid-reactive glass powder obtained by the production method of the present invention is used in a dental glass ionomer cement composition, the acid-reactive glass powder is preferably contained in an amount of 60.0 to 80.0% by mass based on the total composition. When the content of the acid-reactive glass powder is less than 60.0% by mass, mechanical properties may be deteriorated. In addition, when the content of the acid-reactive glass powder exceeds 80.0% by mass, the working time may be shortened, or a homogeneous cement mixture may not be obtained and the mechanical properties may be deteriorated.

In the dental glass ionomer cement composition of the present invention, an acid-reactive glass powder obtained by a conventional production method may be mixed for the purpose of adjusting mechanical properties and setting property as long as various properties are not adversely affected. In this case, the mixing ratio of the acid-reactive glass powder obtained by the production method of the present invention to the acid-reactive glass powder obtained by the conventional production method is preferably in the range of 50 : 50 to 99 : 1 by mass ratio.

The dental glass ionomer cement composition comprising the acid-reactive glass powder obtained by the production method of the present invention (hereinafter referred to as the dental glass ionomer cement composition of the present invention) further comprises a polyalkenoic acid, water, and the like. The respective components are described in detail below.

As the polyalkenoic acid that can be used in the dental glass ionomer cement composition of the present invention, any homopolymer or copolymer of an alkenoic acid at least having one or more carboxy groups in the molecule, such as an unsaturated monocarboxylic acid, an unsaturated dicarboxylic acid, an unsaturated tricarboxylic acid or the like can be used without any limitation. Furthermore, even when the polyalkenoic acid is a copolymer of a polymerizable monomer having no acidic group in the molecule and an alkenoic acid, there are not any problems.

Examples of the alkenoic acid that can be used for obtaining the polyalkenoic acid specifically include, but are not limited to, acrylic acid, methacrylic acid, 2-chloroacrylic acid, 3-chloroacrylic acid, 2-cyanoacrylic acid, aconitic acid, mesaconic acid, maleic acid, maleic anhydride, itaconic acid, itaconic anhydride, fumaric acid, glutaconic acid, citraconic acid, utraconic acid, 1-butene-1,2,4-tricarboxylic acid, and/or 3-butene-1,2,3-tricarboxylic acid. Among these, it is preferable to use a polyalkenoic acid synthesized by using only acrylic acid as a starting material, or a polyalkenoic acid synthesized by using two or more starting materials, such as acrylic acid and maleic acid, acrylic acid and maleic anhydride, acrylic acid and itaconic acid, or acrylic acid and 3-butene-1,2,3-tricarboxylic acid, and the like. As the polyalkenoic acid that can be used in the dental glass ionomer cement composition of the present invention, a commercially available polyalkenoic acid can be used without any problems.

The polymerization method to be used to obtain various polyalkenoic acids is not particularly limited, and polyalkenoic acids polymerized by any of such methods as solution polymerization, suspension polymerization, and emulsion polymerization can be used without any limitations. The polymerization initiator and the chain transfer agent to be used in the synthesis of the polymer may be appropriately selected in order to obtain a desired polymer. Such polyalkenoic acids thus obtained may be used alone or in combination of several polyalkenoic acids.

The polyalkenoic acid is preferably contained in an amount of 5.0 to 25.0% by mass based on the total dental glass ionomer cement composition of the present invention. When the content of the polyalkenoic acid is less than 5.0% by mass, the mechanical properties may be deteriorated. When the content of the polyalkenoic acid is more than 25.0% by mass, the working time may be shortened, or a homogeneous cement mixture may not be obtained and the mechanical properties may be deteriorated.

Water that can be used in the dental glass ionomer cement composition of the present invention functions as a solvent for dissolving the polyalkenoic acid, and is also an essential component for diffusing metal ions released from the acid-reactive glass powder and inducing a crosslinking reaction with the polyalkenoic acid.

Any water that contains no impurities that adversely affect the setting property and the mechanical properties of the dental glass ionomer cement composition of the present invention can be used without any limitations. Specifically, distilled water or ion-exchanged water is preferably used.

Water is preferably contained in an amount of 10.0 to 25.0% by mass based on the total dental glass ionomer cement composition of the present invention. When the water content is less than 10.0% by mass, the working time may be shortened, or a homogeneous cement mixture may not be obtained and the mechanical properties may be deteriorated. When the content of water is more than 25.0% by mass, the mechanical properties may be deteriorated.

In the dental glass ionomer cement composition of the present invention, an acidic compound may be optionally incorporated for the purpose of adjusting the working time and the setting time. Examples of the acidic compound specifically include, but are not limited thereto, carboxylic acid compounds such as tartaric acid, citric acid, maleic acid, fumaric acid, malic acid, aconitic acid, tricarballylic acid, itaconic acid, 1-butene-1,2,4-tricarboxylic acid, 3-butene-1,2,3-tricarboxylic acid and the like; phosphoric acid compounds such as phosphoric acid, pyrophosphoric acid, tripolyphosphoric acid and/or the like; and metal salts of these acidic compounds. These acidic compounds may be used alone or in combination of several acidic compounds.

In addition, the dental glass ionomer cement composition of the present invention may optionally contain such components as conventionally known additives including a non-acid-reactive powder, a surfactant, an antiseptic agent, an antibacterial agent, a colorant, a fluorescent agent, an inorganic fiber material, an organic fiber material and the like, as necessary.

Examples of the form of the dental glass ionomer cement composition of the present invention include, but are not limited to: a combination of a powder comprising an acid-reactive glass powder and a liquid comprising a polyalkenoic acid and water, a combination of a powder comprising an acid-reactive glass powder and a polyalkenoic acid and a liquid comprising water, and a combination of a powder comprising an acid-reactive glass powder and a polyalkenoic acid and a liquid comprising a polyalkenoic acid and water. The proportion in these combinations is not particularly limited, but the mass ratio of a powder to a liquid (powder/liquid) is preferably in the range of 0.5/1.0 to 8.0/1.0, more preferably in the range of 1.0/1.0 to 6.0/1.0, and still more preferably in the range of 2.0/1.0 to 4.0/1.0.

The dental glass ionomer cement composition of the present invention can be used not only as a filling material and a luting material but also in a wide range of applications in dental treatment, such as a pit and fissure sealant material, a base/lining material, a core build-up material and the like.

### EXAMPLES

Hereinafter, the present invention is described specifically by way of Examples, but the present invention is not limited by these Examples. The test methods for evaluating the performance of the dental glass ionomer cement compositions used in Examples and Comparative Examples are as follows.

### [Mixing property]

Under an environment with a temperature of 23°C and a humidity of 50%, a dental glass ionomer cement composition of the present invention was mixed at a ratio described in an Example (the amount of the liquid was 0.1 g), and the mixing property at that time was evaluated according to the following criteria.

In the present specification, the evaluation was performed by five testers, and when the average value was 3 or more, it was determined that good mixing property was exhibited.

### [Evaluation criteria]

5: Mixing was able to be easily completed. Specifically, the mixing was able to be completed in less than 15 seconds.

4: Mixing was able to be completed. Specifically, the mixing was able to be completed in 10 seconds or more and less than 20 seconds.

3: Although the viscosity of the cement mixture was slightly high, the mixing was successfully completed without problems. Specifically, the mixing was able to be completed in 20 seconds or more and less than 30 seconds.

2: The viscosity of the cement mixture was high, and the mixing was difficult. Specifically, a mixing time of 30 seconds or more was needed. Alternatively, during the mixing, a setting reaction partially proceeded, and it was difficult to make the cement mixture into one mass.

1: Since a large number of coarse particles were contained, a crunchy feeling like sand was felt during mixing, and it was impossible to obtain a smooth and homogeneous cement mixture.

### [Working time]

A dental glass ionomer cement composition of the present invention was mixed in a ratio described in an Example under an environment with a temperature of 23°C and a humidity of 50%, and then the fluidity of the resulting cement mixture was examined with a spatula. With the start of the mixing taken as a base point, the time taken until the fluidity of the cement mixture was lost and it became impossible to perform filling and creating the shape was taken as the working time. In the present specification, the evaluation was performed once by each of five testers, and the average value thereof was taken as the working time. When the working time was 1.5 minutes or more, it was determined that the tested composition had a sufficient working time.

### [Compressive strength]

Compressive strength was measured by the following procedure in accordance with ISO 9917-1:2007. A cement mixture was filled in a stainless steel mold (column shape having an inner diameter of 4 mm and a height of 6 mm) under an environment with a temperature of 23°C and a humidity of 50%, and then left at rest in a thermostatic and humidifying chamber with a temperature of 37°C and a humidity of 100%. After being left at rest for 1 hour, the set cement was removed from the mold and used as a test specimen. The test specimen was immersed in ion-exchanged water at 37°C for 24 hours since the completion of the mixing, and then the compressive strength of the test specimen was measured at a crosshead speed of 1 mm/min using an Instron universal testing machine (model: 5567A). When the compressive strength was 180 MPa or more, it was determined that the test specimen had sufficient compressive strength.

As a result of evaluating the above-mentioned performance, one exhibiting good mixing property, a sufficient working time, and a high compressive strength was determined to be one having desirable characteristics as a dental glass ionomer cement composition.

The methods for producing dental glass ionomer cement compositions used in Examples and Comparative Examples are described below.

### [Production of acid-reactive glass powder]

### [Production of acid-reactive glass powders 1 to 7 (G1 to G7)]

Silica, aluminum oxide, aluminum phosphate, aluminum fluoride, and strontium carbonate were mixed in the proportions of 23% by mass, 8% by mass, 13% by mass, 14% by mass, and 42% by mass, respectively, and then melted, and the melt was quenched in water, to obtain a glass. The resulting glass was pulverized for arbitrary periods of time with a roll crusher (Double Roll Crusher 1022-AFS: manufactured by Yoshida Seisakusho Co., Ltd.) and/or a vibration mill (MB-100: manufactured by Chuo Kakohki Co., Ltd.), to obtain acid-reactive glass powders 1 to 7 (G1 to G7) each having a 50% particle size in the range of 0.9 to 7 µm.

### [Measurement of particle size distribution of acid-reactive glass powders 1 to 7 (G1 to G7)]

The 50% particle size of an acid-reactive glass powder was measured with a laser diffraction particle size analyzer (Microtrack MT3300EXII: manufactured by Microtrack Bell Corp.) equipped with a wet disperser. Specifically, 0.05 g to 0.30 g of the acid-reactive glass powder after the pulverising was put into a sample conditioner installed in the apparatus and mixed with ion-exchanged water, and then subjected to ultrasonic dispersion treatment for 10 minutes. Thus, a dispersion of glass particles was prepared. This dispersion was subjected to measurement with the analyzer set as follows: the number of measurements: 2 times/average, measurement time: 30 seconds, particle refractive index: 1.55, particle shape: non-spherical, sensitivity setting: standard, distribution expression: volume, upper limit of measurement: 2000 µm, lower limit of measurement: 0.021 µm, particle transparency: transparent, and solvent refractive index: 1.33. As a result of the particle size distribution measurement, the particle size distributions of the acid-reactive glass powders 1 to 7 (G1 to G7) represented the values shown in Table 1.

**[Table 1]**

| Acid-reactive glass powders 1 to 7 (G1 to G7) | | | | | | | |
|---|---|---|---|---|---|---|---|
| | G1 | G2 | G3 | G4 | G5 | G6 | G7 |
| 50% Particle size /µm | 2.5 | 1 | 5 | 3 | 7 | 0.9 | 2 |

### [Production of acid-reactive glass powders 8 to 13 (G8 to G13)]

Acid-reactive glass powders were produced by the same method as that used for the acid-reactive glass powders 1 to 7 (G1 to G7) except that the particle size was adjusted to the 50% particle sizes as shown in Table 2 by modifying the secondary crusher, the pulverizing machine, and/or the pulverizing time.

**[Table 2]**

| Acid-reactive glass powders 8 to 13 (G8 to G13) | | | | | | |
|---|---|---|---|---|---|---|
| | G8 | G9 | G10 | G11 | G12 | G13 |
| 50% Particle size /µm | 1000 | 250 | 3000 | 4000 | 200 | 11 |

### [Production of acid-reactive glass powders 14 to 31 (G14 to G31)]

In accordance with Table 3, the acid-reactive glass powders 8 to 12 (G8 to G12) shown in Table 2 were pulverized in a state where the acid-reactive glass powders 1 to 7 (G1 to G7) shown in Table 1 were made to coexist, to obtain the acid-reactive glass powders 14 to 29 (G14 to G29) shown in Table 4.

In addition, 50 parts of the acid-reactive glass powder 1 (G1) shown in Table 1 and 50 parts of the acid-reactive glass powder 13 (G 13) shown in Table 2 were mixed in a tumbler mixer for 60 minutes without being pulverized, to obtain acid-reactive glass powder 30 (G30) shown in Table 4.

Further, 50 parts of the acid-reactive glass powder 1 (G1) shown in Table 1 and 50 parts of the acid-reactive glass powder 9 (G9) shown in Table 2 were mixed in a tumbler mixer for 60 minutes without being pulverized, to obtain acid-reactive glass powder 31 (G31) shown in Table 4.

A typical particle size distribution diagram is shown in Fig. 4.

**[Table 3]**

| Production conditions for acid-reactive glass powders 14 to 29 (G14 to G29) | | | | | | | | | | | | | | | | |
|---|---|---|---|---|---|---|---|---|---|---|---|---|---|---|---|---|
| | G14 | G15 | G16 | G17 | G18 | G19 | G20 | G21 | G22 | G23 | G24 | G25 | G26 | G27 | G28 | G29 |
| Small-particle-size acid-reactive glass powder | G4 | G1 | G2 | G3 | G3 | G1 | G1 | G4 | G2 | G3 | G1 | G4 | G5 | G6 | G5 | G7 |
| (50% Particle size/µm) | 3 | 2.5 | 1 | 5 | 5 | 2.5 | 2.5 | 3 | 1 | 5 | 2.5 | 3 | 7 | 0.9 | 7 | 2 |
| Large-particle-size acid-reactive glass powder | G9 | G8 | G9 | G10 | G9 | G8 | G10 | G8 | G9 | G10 | G11 | G12 | G8 | G8 | G10 | G9 |
| (50% Particle size/µm) | 250 | 1000 | 250 | 3000 | 250 | 1000 | 3000 | 1000 | 250 | 3000 | 4000 | 200 | 1000 | 1000 | 3000 | 250 |
| Mixing ratio (mass ratio) of small-particle-size acid-reactive glass powder to large-particle-size acid-reactive glass powder | 50:50 | 50:50 | 90:10 | 10:90 | 90:10 | 80:20 | 20:80 | 90:10 | 60:40 | 20:80 | 30:70 | 60:40 | 70:30 | 60:40 | 50:50 | 90:10 |

**[Table 4]**

| Acid-reactive glass powders 14 to 31 (G14 to G31) | | | | | | | | | | | | | | | | | | |
|---|---|---|---|---|---|---|---|---|---|---|---|---|---|---|---|---|---|---|
| | G14 | G15 | G16 | G17 | G18 | G19 | G20 | G21 | G22 | G23 | G24 | G25 | G26 | G27 | G28 | G29 | G30 | G31 |
| 10% Particle size /µm | 0.8 | 1.2 | 0.9 | 3.0 | 1.1 | 1.0 | 2.5 | 1.0 | 1.2 | 2.6 | 2.2 | 0.8 | 2.4 | 0.9 | 2.7 | 1.1 | 1.7 | 1.5 |
| 50% Particle size /µm | 2.9 | 3.8 | 2.0 | 10.0 | 3.2 | 3.5 | 7.0 | 3.3 | 2.2 | 10.0 | 6.8 | 3.1 | 7.8 | 2.8 | 11.5 | 1.8 | 6.0 | 113.8 |
| 90% Particle size /µm | 22.4 | 15.3 | 9.9 | 21.0 | 11.0 | 14.2 | 20.0 | 13.2 | 9.5 | 23.0 | 18.0 | 8.5 | 17.3 | 10.5 | 22.2 | 7.8 | 17.6 | 427.6 |
| Position of first peak or shoulder (P1) /µm | 1.9 | 2.5 | 1.0 | 5.0 | 2.2 | 2.0 | 4.1 | 1.9 | 2.2 | 4.2 | 3.8 | 1.5 | 5.0 | 0.9 | 6.2 | 1.5 | 4.1 | 2.3 |
| Position of second peak or shoulder (P2) /µm | 14.3 | 8.3 | 3.0 | 17.0 | 4.1 | 9.1 | 16.0 | 8.5 | 4.4 | 20.0 | 13.0 | 3.3 | 11.0 | 4.5 | 12.5 | 4.0 | 15.2 | 352.0 |
| Difference between P1 and P2 /µm | 12.4 | 5.8 | 2.0 | 12.0 | 1.9 | 7.1 | 11.9 | 6.6 | 2.2 | 15.8 | 9.2 | 1.8 | 6.0 | 3.6 | 6.3 | 2.5 | 11.1 | 349.7 |
| Frequency of P1 /% | 3.1 | 3.2 | 4.0 | 2.5 | 3.3 | 4.5 | 2.7 | 4.5 | 3.6 | 2.7 | 2.5 | 2.2 | 3.2 | 3.7 | 2.7 | 4.2 | 3.2 | 2.3 |
| Frequency of P2 /% | 1.4 | 2.4 | 2.1 | 2.5 | 2.2 | 0.9 | 3.0 | 0.8 | 2.7 | 2.2 | 3.5 | 3.7 | 2.6 | 2.4 | 2.5 | 1.1 | 2.6 | 6.1 |
| Ratio of frequency of P1 to P2 (P1/P2) | 2.2 | 1.3 | 1.9 | 1.0 | 1.5 | 5.0 | 0.9 | 5.6 | 1.3 | 1.2 | 0.7 | 0.6 | 1.2 | 1.5 | 1.1 | 3.8 | 1.2 | 0.4 |
| Difference between 10% particle size and 90% particle size /µm | 21.6 | 14.1 | 9.0 | 18.0 | 9.9 | 13.2 | 17.5 | 12.2 | 8.3 | 20.4 | 15.8 | 7.7 | 14.9 | 9.6 | 19.5 | 6.7 | 15.9 | 426.1 |

### [Polyalkenoic acid]

PCA1: acrylic acid-3-butene-1,2,3-tricarboxylic acid copolymer powder (weight average molecular weight: 150,000)
PCA2: acrylic acid homopolymer powder (weight average molecular weight: 100,000)

### [Water]

IEW: ion-exchanged water

### [Other components]

TA: tartaric acid

### [Preparation of powder and liquid]

The compositions of the powders P1 to P 20 are shown in Table 5. As powders P1 to P6 and P8 to P20, the acid-reactive glass powders shown in Tables 2 to 4 were used as received. Powder P7 was prepared by mixing the respective components in the proportions shown in Table 5. The liquids L1 to L4 were prepared by mixing the respective components in the proportions shown in Table 6.

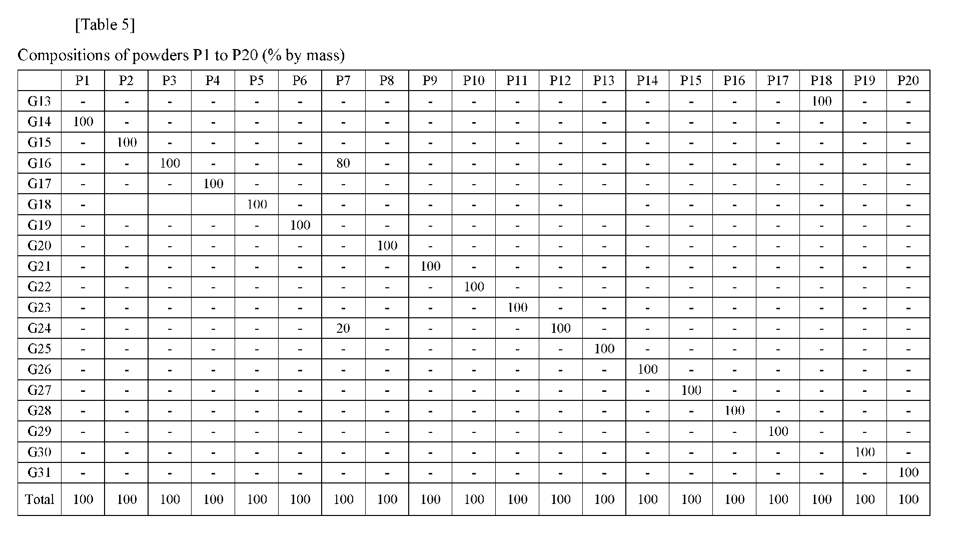

**[Table 6]**

| Composition of liquid (% by mass) | | | | |
|---|---|---|---|---|
| | L1 | L2 | L3 | L4 |
| PCA1 | 45.0 | - | 60.0 | 30.0 |
| PCA2 | - | 40.0 | - | - |
| IEW | 50.0 | 50.0 | 38.0 | 60.0 |
| TA | 5.0 | 10.0 | 2.0 | 10.0 |
| Total | 100.0 | 100.0 | 100.0 | 100.0 |

For the dental glass ionomer cement compositions (Examples 1 to 21, Comparative Examples 1 to 10) obtained by mixing the powders and the liquids according to the combinations and the powder/liquid ratios shown in Examples and Comparative Examples in Tables 7 to 10, the mixing property, the working time, and the compressive strength were evaluated in accordance with the above-described methods. The results are shown in Tables 7 to 10.

**[Table 7]**

| Combinations and evaluation results of Examples 1 to 8 | | | | | | | | | |
|---|---|---|---|---|---|---|---|---|---|
| | | Example 1 | Example 2 | Example 3 | Example 4 | Example 5 | Example 6 | Example 7 | Example 8 |
| Powder | | P1 | P2 | P3 | P4 | P5 | P6 | P7 | P8 |
| Liquid | | L1 | L1 | L1 | L1 | L1 | L1 | L1 | L1 |
| Powder/liquid ratio * 1 | | 3.0/1.0 | 3.0/1.0 | 3.0/1.0 | 3.5/1.0 | 3.0/1.0 | 2.0/1.0 | 3.0/1.0 | 3.0/1.0 |
| Acid-reactive glass powder (% by mass) | G14 | 74.9 | - | - | - | - | - | - | - |
| | G15 | - | 74.9 | - | - | - | - | - | - |
| | G16 | - | - | 74.9 | - | - | - | 59.9 | - |
| | G17 | - | - | - | 77.8 | - | - | - | - |
| | G18 | - | - | - | - | 74.9 | - | - | - |
| | G19 | - | - | - | - | - | 66.6 | - | - |
| | G20 | - | - | - | - | - | - | - | 74.9 |
| | G24 | - | - | - | - | - | - | 15.0 | - |
| Polyalkenoic acid (% by mass) | PCA1 | 11.3 | 11.3 | 11.3 | 10.0 | 11.3 | 15.0 | 11.3 | 11.3 |
| Water (% by mass) | IEW | 12.5 | 12.5 | 12.5 | 11.1 | 12.5 | 16.7 | 12.5 | 12.5 |
| Other components (% by mass) | TA | 1.3 | 1.3 | 1.3 | 1.1 | 1.3 | 1.7 | 1.3 | 1.3 |
| Total (% by mass) | | 100.0 | 100.0 | 100.0 | 100.0 | 100.0 | 100.0 | 100.0 | 100.0 |
| Mixing property | | 4.6 | 4.4 | 4.0 | 4.8 | 4.6 | 4.4 | 4.6 | 4.6 |
| Working time/min | | 2.6 | 2.4 | 2.2 | 2.8 | 2.0 | 3.0 | 2.4 | 2.3 |
| Compressive strength/MPa | | 255 | 254 | 262 | 251 | 249 | 237 | 218 | 220 |

| | | | | | | | | | |
|---|---|---|---|---|---|---|---|---|---|
| ^{∗}1 Powder/liquid ratio: mass ratio of powder to liquid in mixing (mass of powder/mass of liquid) | | | | | | | | | |

**[Table 8]**

| Combinations and evaluation results of Examples 9 to 14 | | | | | | | |
|---|---|---|---|---|---|---|---|
| | | Example 9 | Example 10 | Example 11 | Example 12 | Example 13 | Example 14 |
| Powder | | P9 | P10 | P11 | P2 | P3 | P4 |
| Liquid | | L1 | L1 | L1 | L2 | L3 | L4 |
| Powder/liquid ratio * 1 | | 3.0/1.0 | 3.0/1.0 | 3.0/1.0 | 2.5/1.0 | 2.5/1.0 | 2.5/1.0 |
| Acid-reactive glass powder (% by mass) | G15 | - | - | - | 71.4 | - | - |
| | G16 | - | - | - | - | 71.4 | |
| | G17 | - | - | - | - | - | 71.4 |
| | G21 | 74.9 | - | - | - | - | - |
| | G22 | - | 74.9 | - | - | - | - |
| | G23 | - | - | 74.9 | - | - | - |
| Polyalkenoic acid (% by mass) | PCA1 | 11.3 | 11.3 | 11.3 | - | 17.1 | 8.6 |
| | PCA2 | - | - | - | 11.4 | - | - |
| Water (% by mass) | IEW | 12.5 | 12.5 | 12.5 | 14.3 | 10.9 | 17.1 |
| Other components (% by mass) | TA | 1.3 | 1.3 | 1.3 | 2.9 | 0.6 | 2.9 |
| Total (% by mass) | | 100.0 | 100.0 | 100.0 | 100.0 | 100.0 | 100.0 |
| Mixing property | | 3.4 | 3.4 | 4.6 | 4.8 | 3.8 | 4.8 |
| Working time/min | | 1.9 | 1.8 | 2.5 | 2.7 | 2.3 | 3.0 |
| Compressive strength/MPa | | 250 | 246 | 210 | 240 | 238 | 221 |

| | | | | | | | |
|---|---|---|---|---|---|---|---|
| ^{∗}1 Powder/liquid ratio: mass ratio of powder to liquid in mixing (mass of powder/mass of liquid) | | | | | | | |

**[Table 9]**

| Combinations and evaluation results of Examples 15 to 21 | | | | | | | | |
|---|---|---|---|---|---|---|---|---|
| | | Example 15 | Example 16 | Example 17 | Example 18 | Example 19 | Example 20 | Example 21 |
| Powder | | P3 | P4 | P4 | P3 | P4 | P5 | P2 |
| Liquid | | L1 | L1 | L4 | L1 | L4 | L3 | L4 |
| Powder/liquid ratio *1 | | 1.5/1.0 | 4.0/1.0 | 5.0/1.0 | 0.7/1.0 | 6.0/1.0 | 1.4/1.0 | 1.4/1.0 |
| Acid-reactive glass powder (% by mass) | G15 | - | - | - | - | - | - | 58.3 |
| | G16 | 60.0 | - | - | 41.2 | - | - | - |
| | G17 | - | 80.0 | 83.3 | - | 85.7 | - | - |
| | G18 | - | - | - | - | - | 58.4 | - |
| Polyalkenoic acid (% by mass) | PCA1 | 18.0 | 9.0 | 5.0 | 26.5 | 4.3 | 25.0 | 12.5 |
| Water (% by mass) | IEW | 20.0 | 10.0 | 10.0 | 29.4 | 8.6 | 15.8 | 25.0 |
| Other components (% by mass) | TA | 2.0 | 1.0 | 1.7 | 2.9 | 1.4 | 0.8 | 4.2 |
| Total (% by mass) | | 100.0 | 100.0 | 100.0 | 100.0 | 100.0 | 100.0 | 100.0 |
| Mixing property | | 4.8 | 4.2 | 3.4 | 4.8 | 3.2 | 3.4 | 4.8 |
| Working time/min | | 3.5 | 2.2 | 1.8 | 4.0 | 1.5 | 1.7 | 2.3 |
| Compressive strength/MPa | | 208 | 240 | 218 | 200 | 209 | 222 | 191 |

| | | | | | | | | |
|---|---|---|---|---|---|---|---|---|
| ^{∗}1 Powder/liquid ratio: mass ratio of powder to liquid in mixing (mass of powder/mass of liquid) | | | | | | | | |

**[Table 10]**

| Combinations and evaluation results of Comparative Examples 1 to 10 | | | | | | | | | | | |
|---|---|---|---|---|---|---|---|---|---|---|---|
| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
| Powder | | P12 | P13 | P14 | P15 | P16 | P17 | P18 | P18 | P19 | P20 |
| Liquid | | L1 | L1 | L1 | L1 | L2 | L2 | L2 | L3 | L1 | L1 |
| Powder/liquid ratio * 1 | | 2.5/1.0 | 2.5/1.0 | 2.5/1.0 | 2.5/1.0 | 3.0/1.0 | 3.0/1.0 | 3.0/1.0 | 3.0/1.0 | 3.0/1.0 | 3.0/1.0 |
| Acid-reactive glass powder (% by mass) | G14 | - | - | - | - | - | - | 75.0 | 75.0 | - | - |
| | G24 | 71.4 | - | - | - | - | - | - | - | - | - |
| | G25 | - | 71.4 | - | - | - | - | - | - | - | - |
| | G26 | - | - | 71.4 | - | - | - | - | - | - | - |
| | G27 | - | - | - | 71.4 | - | - | - | - | - | - |
| | G28 | - | - | - | - | 75.0 | - | - | - | - | - |
| | G29 | - | - | - | - | - | 75.0 | - | - | - | - |
| | G30 | - | - | - | - | - | - | - | - | 74.9 | - |
| | G31 | - | - | - | - | - | - | - | - | - | 74.9 |
| Polyalkenoic acid (% by mass) | PCA1 | 12.9 | 12.9 | 12.9 | 12.9 | - | - | - | 15.0 | 11.3 | 11.3 |
| | PCA2 | - | - | - | - | 10.0 | 10.0 | 10.0 | - | - | - |
| Water (% by mass) | IEW | 14.3 | 14.3 | 14.3 | 14.3 | 12.5 | 12.5 | 12.5 | 9.5 | 12.5 | 12.5 |
| Other components (% by mass) | TA | 1.4 | 1.4 | 1.4 | 1.4 | 2.5 | 2.5 | 2.5 | 0.5 | 1.3 | 1.3 |
| Total (% by mass) | | 100.0 | 100.0 | 100.0 | 100.0 | 100.0 | 100.0 | 100.0 | 100.0 | 100.0 | 100.0 |
| Mixing property | | 3.4 | 2.4 | 3.4 | 2.4 | 3.6 | 2.2 | 3.4 | 2.6 | 2.2 | 1.0 |
| Working time/min | | 2.0 | 1.2 | 2.2 | 1.4 | 2.2 | 1.3 | 1.0 | 1.3 | 2.3 | 3.8 |
| Compressive strength/MPa | | 161 | 208 | 155 | 211 | 159 | 214 | 143 | 151 | 170 | 128 |

| | | | | | | | | | | | |
|---|---|---|---|---|---|---|---|---|---|---|---|
| ^{∗}1 Powder/liquid ratio: mass ratio of powder to liquid in mixing (mass of powder/mass of liquid) | | | | | | | | | | | |

### <Examples 1 to 21>

Example 1 exhibited good mixing property and had a sufficient working time. It also exhibited higher compressive strength and had properties desirable as a dental glass ionomer cement.

Examples 2 to 21 are compositions obtained by modifying the method for producing an acid-reactive glass powder, the type of the polyalkenoic acid, the polyalkenoic acid concentration in the liquid, and/or the powder/liquid ratio from Example 1. As a result of evaluating Examples 2 to 21, all of them exhibited good mixing property and had sufficient working times. It also exhibited higher compressive strength and had properties desirable as a dental glass ionomer cement.

### <Comparative Example 1>

As to the acid-reactive glass powder used in Comparative Example 1, the large-particle-size acid-reactive glass powder used in the production thereof had a particle size as large as 4000 µm. In the particle size distribution of the obtained acid-reactive glass powder, the ratio (P1/P2) of the frequency of the first peak (P1) to the second peak (P2) was as small as 0.7. As a result of evaluating Comparative Example 1, it was found that the mixing property was poor and the compressive strength was low.

### <Comparative Example 2>

As to the acid-reactive glass powder used in Comparative Example 2, the large-particle-size acid-reactive glass powder used in the production thereof had a particle size as small as 200 µm. In the particle size distribution of the obtained acid-reactive glass powder, the difference (difference in particle size) between the first peak (P1) and the second shoulder (P2) was as small as 1.8 µm. Furthermore, the ratio (P1/P2) of the frequency of the first peak (P1) to the frequency of the second shoulder (P2) was as small as 0.6, and the difference between the 10% particle size and the 90% particle size was as small as 7.7 µm. As a result of evaluating Comparative Example 2, it was found that the mixing property was poor and the working time was short.

### <Comparative Example 3>

As to the acid-reactive glass powder used in Comparative Example 3, the small-particle-size acid-reactive glass powder used in the production thereof had a particle size as large as 7 µm. As a result of evaluating Comparative Example 3, it was found that the compressive strength was low.

### <Comparative Example 4>

As to the acid-reactive glass powder used in Comparative Example 4, the small-particle-size acid-reactive glass powder used in the production thereof had a particle size as small as 0.9 µm. In the particle size distribution of the obtained acid-reactive glass powder, the position of the first peak (P1) was as small as 0.9 µm. As a result of evaluating Comparative Example 4, it was found that the mixing property was poor and the working time was short.

### <Comparative Example 5>

As to the acid-reactive glass powder used in Comparative Example 5, the small-particle-size acid-reactive glass powder used in the production thereof had a particle size as large as 7 µm. In the particle size distribution of the obtained acid-reactive glass powder, the 50% particle size was as large as 11.5 µm. Furthermore, the position of the first peak (P1) was as large as 6.2 µm, and the difference between the 10% particle size and the 90% particle size was as large as 19.5 µm. As a result of evaluating Comparative Example 5, it was found that the compressive strength was low.

### <Comparative Example 6>

As to the acid-reactive glass powder used in Comparative Example 6, in the particle size distribution of the obtained acid-reactive glass powder, the 50% particle size was as small as 1.8 µm, and the difference between the 10% particle size and the 90% particle size was as small as 6.7 µm. As a result of evaluating Comparative Example 6, it was found that the mixing property was poor and the working time was short.

### <Comparative Example 7>

The acid-reactive glass powder used in Comparative Example 7 is one obtained by a conventional production method. Specifically, it is an acid-reactive glass powder having a 50% particle size of 11 µm and obtained not by pulverizing a large-particle-size acid-reactive glass powder in a state where a small-particle-size acid-reactive glass powder was made to coexist but by pulverizing glass only single in the type of glass constituent and in the particle size merely with a roll crusher and a ball mill. As a result of evaluating Comparative Example 7, it was found that the working time was short and the compressive strength was low.

### <Comparative Example 8>

The acid-reactive glass powder used in Comparative Example 8 is one obtained by a conventional production method. Specifically, it is an acid-reactive glass powder having a 50% particle size of 11 µm and obtained not by pulverizing a large-particle-size acid-reactive glass powder in a state where a small-particle-size acid-reactive glass powder was made to coexist but by pulverizing glass only single in the type of glass constituent and in the particle size merely for an arbitrary time. As a result of evaluating Comparative Example 8, it was found that the mixing property was poor and the working time was short. Furthermore, it was found that the compressive strength was low.

### <Comparative Example 9>

The acid-reactive glass powder used in Comparative Example 9 is an acid-reactive glass powder having a 50% particle size of 6.0 µm and obtained by merely mixing, without pulverizing, an acid-reactive glass powder having a 50% particle size of 2.5 µm and obtained by a conventional production method, and an acid-reactive glass powder having a 50% particle size of 11 µm and obtained by a conventional production method. As a result of evaluating Comparative Example 9, it was found that the mixing property was poor and the compressive strength was low.

### <Comparative Example 10>

The acid-reactive glass powder used in Comparative Example 10 is an acid-reactive glass powder having a 50% particle size of 113.8 µm and obtained by merely mixing, without pulverizing, an acid-reactive glass powder having a 50% particle size of 2.5 µm and obtained by a conventional production method, and an acid-reactive glass powder having a 50% particle size of 250 µm and obtained by a conventional production method. As a result of evaluating Comparative Example 10, it was found that the mixing property was poor and the compressive strength was low.

### Exemplary embodiments include, but are not limited to, the following.

Embodiment 1: A method for producing an acid-reactive glass powder, the method comprising: pulverizing a large-particle-size acid-reactive glass powder having a range of 250 to 3000 µm of a 50% particle size in a state where the small-particle-size acid-reactive glass powder having a range of 1 to 5 µm of a 50% particle size coexists (or is made present together or is combined) with the large-particle-size acid-reactive glass powder to obtain an acid-reactive glass powder having a range of 2 to 10 µm of a 50% particle size.

Embodiment 2: A method for producing an acid-reactive glass powder, the method comprising: pulverizing an acid-reactive glass powder obtained by making a "small-particle-size acid-reactive glass powder having a range of 1 to 5 µm of a 50% particle size" and a "large-particle-size acid-reactive glass powder having a range of 250 to 3000 µm of a 50% particle size" prepared separately from each other coexist with each other or combining these powders with each other (the resulting acid-reactive glass powder can also be referred to as "coexisting acid-reactive glass powder or combined acid-reactive glass powder") to obtain an acid-reactive glass powder having a range of 2 to 10 µm of a 50% particle size.

Embodiment 3: The method according to Embodiment 1 or 2, wherein a particle size distribution of the acid-reactive glass powder after the pulverization has a first peak or shoulder (P1) in the range of 1 to 5 µm and a second peak or shoulder (P2) in the range of 3 to 20 µm, and
wherein particle sizes of the first peak or shoulder (P1) and the second peak or shoulder (P2) have a relationship of P1 < P2, and a difference between the particle sizes of the first peak or shoulder (P1) and the second peak or shoulder (P2) is 2 µm or more.

Embodiment 4: The method according to Embodiment 1 or 2, wherein a particle size distribution of the acid-reactive glass powder after the pulverization has a first peak (P1) in the range of 1 to 5 µm and a second peak or shoulder (P2) in the range of 3 to 20 µm, and
wherein particle sizes of the first peak (P1) and the second peak or shoulder (P2) have a relationship of P1 < P2, a difference between the particle sizes of the first peak or shoulder (P1) and the second peak or shoulder (P2) is 2 µm or more, and
wherein a ratio (P1/P2) of a frequency of the first peak (P1) to a frequency of the second peak or shoulder (P2) is in the range of 1.0 to 5.0.

Embodiment 5: The method according to any one of Embodiments 1 to 4, wherein, in a particle size distribution of the acid-reactive glass powder after the pulverization, a difference between a 10% particle size and a 90% particle size is in the range of 9 to 25 µm.

Embodiment 6: An acid-reactive glass powder obtainable by the method according to any one of Embodiments 1 to 5.

Embodiment 7: A dental glass ionomer cement composition comprising the acid-reactive glass powder according to Embodiment 6.

Embodiment 8: A dental glass ionomer cement composition comprising:
60.0 to 80.0% by mass of an acid-reactive glass powder,
5.0 to 25.0% by mass of a polyalkenoic acid, and
10.0 to 25.0% by mass of water,
wherein the acid-reactive glass powder is the acid-reactive glass powder according to Embodiment 6.

### Reference numerals

1. Particle size distribution
2a. First peak
2b. Second peak
3a. First shoulder
3b. Second shoulder

## Claims

1. A method for producing an acid-reactive glass powder, the method comprising: pulverizing a large-particle-size acid-reactive glass powder having a range of 250 to 3000 µm of a 50% particle size in a state where a small-particle-size acid-reactive glass powder having a range of 1 to 5 µm of a 50% particle size coexists with the large-particle-size acid-reactive glass powder, and thereby providing an acid-reactive glass powder having a range of 2 to 10 µm of a 50% particle size.

2. The method according to claim 1, wherein a particle size distribution of the acid-reactive glass powder at a point in time after the pulverization has a first peak or shoulder (P1) in a range of 1 to 5 µm and a second peak or shoulder (P2) in a range of 3 to 20 µm, and
wherein particle sizes of the first peak or shoulder (P1) and the second peak or shoulder (P2) have a relationship of P1 < P2, and a difference between the particle sizes of the first peak or shoulder (P1) and the second peak or shoulder (P2) is 2 µm or more.

3. The method according to claim 1, wherein a particle size distribution of the acid-reactive glass powder at a point in time after the pulverization has a first peak (P1) in a range of 1 to 5 µm and a second peak or shoulder (P2) in a range of 3 to 20 µm, and
wherein particle sizes of the first peak (P1) and the second peak or shoulder (P2) have a relationship of P1 < P2, a difference between the particle sizes of the first peak or shoulder (P1) and the second peak or shoulder (P2) is 2 µm or more, and
wherein a ratio (P1/P2) of a frequency of the first peak (P1) to a frequency of the second peak or shoulder (P2) is in a range of 1.0 to 5.0.

4. The method according to any one of claims 1 to 3, wherein, in a particle size distribution of the acid-reactive glass powder at a point in time after the pulverization, a difference between a 10% particle size and a 90% particle size is in a range of 9 to 25 µm.

5. An acid-reactive glass powder obtainable by the method according to any one of claims 1 to 3.

6. An acid-reactive glass powder obtainable by the method according to claim 4.

7. A dental glass ionomer cement composition comprising the acid-reactive glass powder according to claim 5.

8. A dental glass ionomer cement composition comprising the acid-reactive glass powder according to claim 6.

9. A dental glass ionomer cement composition comprising:
60.0 to 80.0% by mass of an acid-reactive glass powder,
5.0 to 25.0% by mass of a polyalkenoic acid, and
10.0 to 25.0% by mass of water,
wherein the acid-reactive glass powder is the acid-reactive glass powder according to claim 5.
